# EUROPÄISCHE PATENTSCHRIFT

(11) **EP 1 210 136 B1**
(45) Veröffentlichungstag und Bekanntmachung des Hinweises auf die Patenterteilung: **25.01.2006**
(21) Anmeldenummer: 00941864.1
(22) Anmeldetag: 18.07.2000
(51) Int. Cl.: A61M 5/145

(54) **VORRICHTUNG ZUR VERABREICHUNG EINES INJIZIERBAREN PRODUKTS**
DEVICE FOR THE ADMINISTRATION OF AN INJECTABLE PRODUCT
DISPOSITIF D'ADMINISTRATION D'UN PRODUIT INJECTABLE

(30) Priorität: 18.08.1999 DE 19939023
(43) Veröffentlichungstag der Anmeldung: 05.06.2002
(73) Patentinhaber: Disetronic Licensing AG, 3401 Burgdorf (CH)
(72) Erfinder: MICHEL, Peter, CH-3400 Burgdorf (CH)
(74) Vertreter: Schwabe - Sandmair - Marx
(86) Internationale Anmeldenummer: PCT/CH2000/000390
(87) Internationale Veröffentlichungsnummer: WO 2001/012250

(56) Entgegenhaltungen:
- DE-A- 19 614 337
- US-A- 4 437 859
- US-A- 5 788 673
- PATENT ABSTRACTS OF JAPAN vol. 018, no. 001 (C-1148), 6. Januar 1994 (1994-01-06) & JP 05 245197 A (KOBAYASHI PHARMACEUT CO LTD;OTHERS: 01), 24. September 1993 (1993-09-24)

## Beschreibung

Die Erfindung betrifft eine Vorrichtung zur Verabreichung eines injizierbaren Produkts.

Injektionsgeräte, beispielsweise Injektionsspritzen oder Injektionspens, wie die Erfindung sie insbesondere, jedoch nicht ausschließlich betrifft, weisen üblicherweise ein Gehäuse auf, in dem eine Ampulle mit dem zu injizierenden Produkt, ein Fördermittel zur Förderung des Produkts aus der Ampulle, ein Antriebsmittel und eine Kopplungseinrichtung aufgenommen sind. Das Fördermittel wird üblicherweise durch einen in der Ampulle verschiebbaren Kolben gebildet. Als Antriebsmittel dient bei einfachen Spritzen die Muskelkraft des Benutzers. Bekannt ist auch die Verwendung von Federelementen, insbesondere von Druckfedern, als Antriebsmittel. Die Kopplungseinrichtung bildet eine Übertragungsstrecke bzw. Antriebsverbindung von dem Antriebsmittel zu dem Fördermittel.

Die bekannten Antriebsmittel, beispielsweise Antriebsfedern, besitzen den Nachteil, dass die von ihnen aufgebrachte Antriebskraft oder Antriebsenergie im Verlaufe ihrer Freisetzung Änderungen unterliegt. Bei Antriebsfedern ändert sich die Antriebsenergie entsprechend der Federcharakteristik. Die Förderrate des Fördermittels folgt solchen Änderungen. Dementsprechend ändert sich im Verlaufe einer Ausschüttung des Produkts die Ausschüttrate entsprechend der sich ändernden Antriebsenergie.

Die US 5,788,673 offenbart ein Infusionssystem für eine Flüssigkeit, das eine Pumpvorrichtung und eine Spritze umfasst. In der Pumpvorrichtung treibt ein Antriebskolben einen Abtriebskolben über ein Fluid an. Eine Drossel hemmt das Fluid, um von einer Kammer, aus der das Fluid mit dem Antriebskolben verdrängt wird, in eine andere Kammer zu laufen. In einer Ausführungsform sind die Kammern axial hintereinander, in einer anderen Ausführungsform nebeneinander angeordnet.

Aufgabe der Erfindung ist es, eine Vorrichtung zur Verabreichung eines injizierbaren Produkts zu schaffen, mit der das Produkt im Verlaufe einer Injektion oder Infusion gleichmäßig ausgeschüttet wird.
Die Erfindung geht von einer Vorrichtung zur Verabreichung eines injizierbaren Produkts aus, die ein Gehäuse, ein von dem Gehäuse aufgenommenes Behältnis für das Produkt, ein Fördermittel, ein Antriebsmittel und eine Übertragungsstrecke bzw. Kopplungseinrichtung, umfasst. Mit dem Fördermittel wird unmittelbar das Produkt aus dem Behältnis gefördert. Das Antriebsmittel liefert die hierfür erforderliche Antriebsenergie, die in der Übertragungsstrecke bis zu dem Fördermittel übertragen wird, derart, dass die Fördereinrichtung zur Ausschüttung des Produkts von dem Antriebsmittel angetrieben wird.

Vorzugsweise sind das Behältnis, das Fördermittel, das Antriebsmittel und die Übertragungsglieder der Übertragungsstrecke in dem Gehäuse angeordnet. Andere Anordnungen sind grundsätzlich jedoch ebenfalls möglich. Bei dem injizierbaren Produkt handelt es sich vorzugsweise um einen medizinischen oder kosmetischen Wirkstoff, insbesondere in Form einer flüssigen Wirkstofflösung. Ein prominentes Beispiel ist Insulin, das mit der Vorrichtung im Rahmen einer Diabetistherapie verabreicht wird. Die Vorrichtung ist vorzugsweise ein Infusionsgerät. Es kann sich aber auch um ein Injektionsgerät handeln. Das Behältnis kann insbesondere als Ampulle ausgebildet sein, wie dies bei bekannten Infusionsgeräten der Fall ist. Das Fördermittel wird vorzugsweise durch einen in dem Behältnis aufgenommenen Kolben gebildet, der zur Ausschüttung von Produkt auf einen Auslass des Behältnisses zu vorgeschoben wird. Anstatt solch eines Kolbens kann das Fördermittel jedoch grundsätzlich durch jede zur Förderung des Produkts geeignete Pumpenart gebildet werden.

Das Antriebsmittel ist seiner Art nach vorzugsweise derart ausgebildet, dass es bei seiner Auslösung eine in sich selbst gespeicherte Energie freisetzt. Durch eine Kopplungseinrichtung wird diese freigesetzte Energie in der Übertragungsstrecke bis zum Fördermittel übertragen, das seinerseits derart angetrieben das Produkt aus dem Behältnis fördert. Das Antriebsmittel wird vorzugsweise durch eine Antriebsfeder gebildet, besonders bevorzugt ist es eine Druckfeder. Grundsätzlich können jedoch auch andere Bauarten von Antriebsmitteln zum Einsatz kommen, z. B. solche, die ein Druckgas bei Ihrer Auslösung freisetzen.

Nach der Erfindung sind in der Übertragungsstrecke von dem Antriebsmittel zu dem Fördermittel, d.h. in der Kopplungseinrichtung, ein Fluidraum für ein inkompressibles Fluid und eine Druckreduziereinrichtung vorgesehen. Der Fluidraum weist dementsprechend eine Antriebsseite, auf die das Antriebsmittel wirkt, und eine Abtriebsseite auf, die auf das Fördermittel wirkt. Sowohl die Antriebsseite als auch die Abtriebsseite können unmittelbar oder erst über weitere Übertragungsglieder mit dem Antriebsmittel bzw. dem Fördermittel verbunden sein. Der Fluidraum ist an seiner Antriebsseite von dem Antriebsmittel mit Druck beaufschlagbar. Der so erzeugte Druck wird von der Druckreduziereinrichtung zu der Abtriebsseite des Fluidraums hin gemindert. Vorzugsweise wird der Druck auf ein Fünftel oder weniger und besonders bevorzugt auf ein Zehntel oder weniger mittels der Druckreduziereinrichtung gemindert. Durch die Druckreduziereinrichtung wird eine Fluidverbindung geschaffen, die einen Fluidfluss von der Antriebsseite in Richtung Abtriebsseite nur verzögert zulässt, so dass im dynamischen Zustand, d. h. während des Antriebs des Fördermittels, an der Antriebsseite ein größerer Fluiddruck als an der Abtriebsseite herrscht.

Die Erfindung ermöglicht den Einsatz eines Antriebsmittels, in dem eine wesentlich größere Energie gespeichert ist als zum Antrieb des Fördermittels und der damit einhergehenden Ausschüttung des Produkts erforderlich wäre. Die bei der Auslösung des Antriebsmittels freigesetzte, vergleichsweise große Antriebsenergie wird durch die erfindungsgemäße Fluidkopplung auf das für die Ausschüttung und Verabreichung erforderliche Maß herabgesetzt. Das Übermaß an Antriebsenergie steht wegen der erfindungsgemäßen Fluidkopplung kontrolliert für den Antrieb des Fördermittels zur Verfügung. Wird als Antriebsmittel eine Antriebsfeder verwendet, wie dies bevorzugt ist, so kann die Federstärke dieses Antriebsmittels erheblich größer sein als im Falle einer direkten Antriebsverbindung mit dem Fördermittel. Insbesondere kann solch eine Antriebsfeder in einem kleineren Bereich ihrer Federkennlinie betrieben werden als dies im Falle einer direkten Kopplung möglich wäre.

Besonders bevorzugt wird durch die Fluidkopplung ein Arbeitshub des Antriebsmittels in einen Arbeitshub des Fördermittels übertragen, der größer als der Arbeitshub des Antriebsmittels ist. Im Falle einer Druck- oder Zugfeder als Antriebsmittel und eines Kolbens als Fördermittel ist der jeweilige Arbeitshub die Dehnung oder Stauchung der Feder und die in Abhängigkeit von diesem Arbeitshub von dem Kolben zurückgelegte Wegstrecke.

Besonders bevorzugt ist das Fördermittel als Kolben ausgebildet und das Antriebsmittel wirkt ebenfalls auf einen Kolben, der im folgenden als Antriebskolben bezeichnet wird. Die Antriebsseite des Fluidraums wird in dieser Ausführung von einer Kolbenfläche des Antriebskolbens gebildet. Die Kolbenfläche des Antriebskolbens ist vorzugsweise größer als eine Kolbenfläche eines Abtriebskolbens, wobei die Kolbenfläche des Abtriebskolbens die Abtriebsseite des Fluidraums bildet.

Durch dieses Verhältnis der beiden Kolbenflächen wird ein Hub des Antriebskolbens in einen demgegenüber größeren Hub des Abtriebskolbens übertragen. Anders ausgedrückt, es ist zur Erzielung eines bestimmten Hubs des Abtriebskolbens ein kleinerer Hub des Antriebskolbens erforderlich. Dementsprechend kurz kann der Arbeitshub des Antriebsmittels gehalten werden. Das Antriebsmittel kann in einem engen Bereich um seinen optimalen Arbeitspunkt betrieben werden. Ferner findet durch die unterschiedlich großen Kolbenflächen eine Kranreduzierung statt. Es wird die von dem Antriebskolben ausgeübte Kraft entsprechend dem Flächenverhältnis von Antriebskolben und Abtriebskolben reduziert. Diese Reduzierung erfolgt zusätzlich zur Kranreduzierung infolge der Druckreduzierung.

Der Abtriebskolben kann das Fördermittel unmittelbar bilden. Vorzugsweise handelt es sich bei dem Abtriebskolben jedoch um einen anderen Kolben.

Der Fluidraum ist in einen die Antriebsseite umfassenden ersten Teilraum und einen die Antriebsseite umfassenden zweiten Teilraum unterteilt. Der erste Teilraum oder der zweite Teilraum ist als Ringkammer zwischen einer äußeren Hülse und einer inneren Hülse und der andere der beiden Teilräume in der inneren Hülse ausgebildet.

In einem besonders bevorzugten Ausführungsbeispiel sind die beiden Teilräume ausschließlich durch ein Kapillarensystem miteinander verbunden, falls auf der Antriebsseite ein höherer Druck als auf der Abtriebsseite des Fluidraums herrscht. Das Kapillarensystem kann durch eine einzige oder auch durch mehrere Kapillaren gebildet werden.

Die Kapillare oder die mehreren Kapillaren ist oder sind vorteilhafterweise möglichst lang. Vorzugsweise beträgt ihre Länge wenigstens 0,5 m. Sind mehrere Kapillaren ausgebildet, so gilt dies vorzugsweise für jede der Kapillaren. Die Durchflussrate ist bei langen Kapillaren weniger von dem Durchmesser der Kapillare abhängig, wie sich ohne weiteres aus dem Gesetz von Hagen-Poiseuille ergibt. Nach dem Gesetz von Hagen-Poiseuille gehen nämlich Schwankungen des Durchmessers aufgrund von Fertigungsungenauigkeiten in der vierten Potenz in die Durchflussrate ein. Mit zunehmender Länge der Kapillare kann jedoch ihr Durchmesser ebenfalls vergrößert werden, wenn die Durchflussrate konstant bleiben soll. Größere Durchmesser sind zum einen von Hause aus einfacher herstellbar als kleinere Durchmesser, und mit zunehmender Größe des Durchmessers schlagen Abweichungen vom Solldurchmesser zunehmend weniger zu Buche. Ferner wird eine möglichst hohe Viskosität des Arbeitsfluids im Fluidraum bevorzugt.

Das Kapillarensystem weist vorzugsweise eine spiralig verlaufende Kapillare oder mehrere solcher Kapillaren auf. In einem bevorzugten Ausführungsbeispiel wird das Kapillarensystem durch eine einzige, spiralige Kapillare gebildet. Eine spiralige Kapillare bringt nicht nur den Vorteil einer großen Länge mit sich, sondern kann auch einfach hergestellt werden. Sie kann insbesondere in Form eines Außen- oder Innengewindes an einer entsprechenden Mantelfläche eines Kapillarenkörpers gebildet sein. Der Kapillarenkörper mit dem Außen- oder Innengewinde wird vorzugsweise in oder auf einen weiteren Körper mit einer glatten Gegenmantelfläche gesteckt, wobei darauf zu achten ist, dass die Gewindegänge des Kapillarenkörpers an der Gegenmantelfläche gegeneinander abgedichtet sind.

Die Erfindung wird nachfolgend anhand eines bevorzugten Ausrührungsbeispiels beschrieben. Es zeigen:
- Figur 1: ein Infusionsgerät in einem Längsschnitt,
- Figur 2: eine Kapillare gemäß Detail I der Figur 1 und
- Figur 3: eine alternative Ausführungsform einer Kapillare.

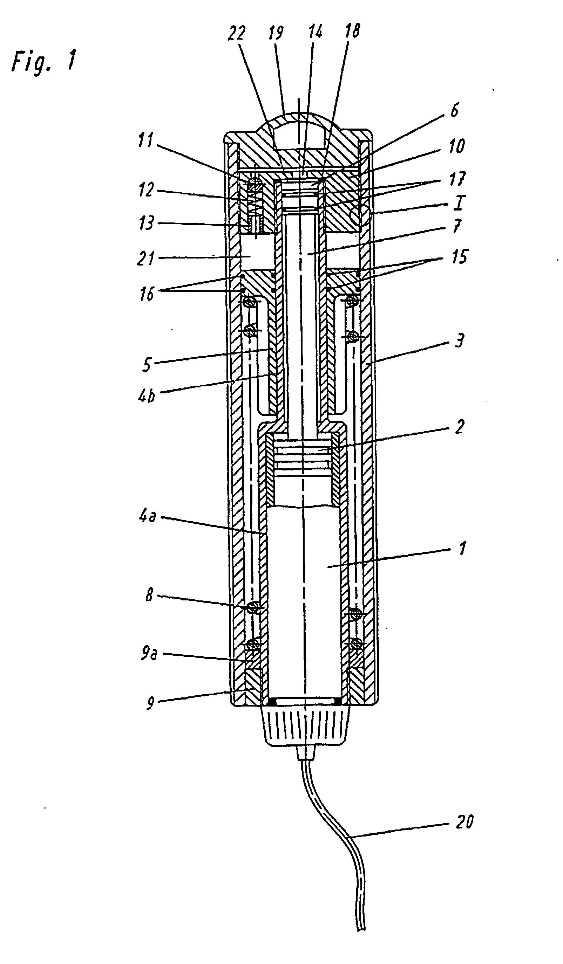

Figur 1 zeigt im Längsschnitt ein Infusionsgerät.

Eine kreiszylindrische äußere Hülse 3 bildet zusammen mit einem Verschlussstück 9 an einem proximalen Ende und einer Verschlusskappe 19 an einem distalen Ende ein Gehäuse des Infusionsgeräts. Ein Behältnishalter 4a ist in einem proximalen Bereich der äußeren Hülse 3 zentriert gehalten. In dem Behältnishalter 4a ist ein Behältnis 1 in Form einer Ampulle ebenfalls zentriert zur Mittellängsachse der äußeren Hülse 3 aufgenommen. Das Behältnis 1 ist mit einem zu injizierenden Produkt, beispielsweise Insulin, gefüllt. In dem Behältnis 1 ist ferner ein Fördermittel 2 in Form eines Förderkolbens geradlinig auf einen Auslass des Behältnisses 1 zu verschiebbar aufgenommen. An den Auslass des Behältnisses 1 ist ein Katheter 20 in an sich bekannter Art und Weise angeschlossen.

In einem distalen Bereich des Infusionsgeräts ist eine innere Hülse 4b konzentrisch zu der äußeren Hülse 3 angeordnet. Im Ausführungsbeispiel sind der Behältnishalter 4a und die innere Hülse 4b als einstückige Hülse ausgebildet. Der Behältnishalter 4a und die innere Hülse 4b könnten auch separate Bauteile sein. Durch die einstückige Ausbildung wird jedoch die gemeinsame, zentrierte Halterung in der äußeren Hülse 3 vereinfacht, wie sich aus Figur 1 und der nachfolgenden Beschreibung ohne weiteres erschließt.

Eine Innenmantelfläche der inneren Hülse 4b bildet eine Gleitführung für einen in der inneren Hülse 4b aufgenommenen Abtriebskolben 6, der mittels einer Kolbenstange 7 mit dem Förderkolben 2 steif verbunden ist. Der Abtriebskolben 6 und die Kolbenstange 7 sind einstückig ausgebildet. Die Kolbenstange 7 stößt an den Förderkolben 2 an. Sie könnte auch fest mit dem Förderkolben verbunden sein; beispielsweise könnte sie mit dem Förderkolben 2 verschraubt sein. Ferner kann die Kolbenstange 7 in einem Halsbereich zwischen dem Behältnishalter 4a und der inneren Hülse 4b ebenfalls gleitgeführt sein, beispielsweise fluiddicht gleitgeführt. Der Abtriebskolben 6 dichtet mit Dichtringen 17 in der Art von Kolbenringen zur inneren Hülse 4b hin ab.

Zwischen der äußeren Hülse 3 und der inneren Hülse 4b ist ein Ringraum gebildet, in dem ein Antriebskolben 5 angeordnet ist. Der Antriebskolben 5 ist ein Ringkolben, der in dem Ringraum zwischen der äußeren Hülse 3 und der inneren Hülse 4b hin und her fluiddicht und eng gleitgeführt wird. In Nuten in einer Innenmantelfläche des Antriebskolbens 5 sind Dichtringe 15 und in Nuten an einer Außenmantelfläche des Antriebskolbens 5 sind weitere Dichtringe 16, jeweils in der Art von Kolbenringen, aufgenommen. Der Antriebskolben 5 weist an einer distalen Stirnseite eine plane Ringfläche auf. In die proximale Richtung verjüngt sich der Antriebskolben 5 zur inneren Hülse 4b hin. Die Verjüngung wird mittels einer Schulter gebildet. Der Schulter liegt in proximaler Richtung gesehen eine Gegenfläche des Infusionsgeräts gegenüber. Die Gegenfläche wird von einem Distanzstück in Form eines Distanzrings 9a gebildet, der den Behältnishalter 4a umgibt und auf dem Verschlussstück 9 lose aufliegt.

In einem Ringraum zwischen der äußeren Hülse 3 einerseits und dem Behältnishalter 4a und der inneren Hülse 4b andererseits ist zwischen den beiden einander gegenüberliegenden Flächen, nämlich der Schulter des Antriebskolbens 5 und dem Distanzring 9a, eine Druckfeder 8 an beide Flächen anstoßend aufgenommen. Durch Variation der Stärke des Distanzrings 9a, d.h. durch Distanzringwechsel, kann die Vorrichtung auf einfache Art und Weise an unterschiedliche Druckfedern 8 angepasst werden, um den Federarbeitsbereich stets optimal einzustellen.

In distaler Richtung ist hinter dem Antriebskolben 5 ein Kapillarenkörper 10 angeordnet. Der Kapillarenkörper 10 weist einen proximalen Ringbereich auf und schließt an seinem distalen Ende mit einem Boden ab. Im Bereich seines Ringkörpers dichtet der Kapillarenkörper 10 gegen die äußere Hülse 3 und vorzugsweise auch gegen die innere Hülse 4b fluiddicht ab. Eine distale Stirnfläche der inneren Hülse 4b drückt über einen Dichtring 18 fluiddicht gegen den Boden des Kapillarenkörpers 10. Im Bereich einer distalen, stirnseitigen Öffnung der inneren Hülse 4b, die von dem Dichtring 18 abgedichtet wird, ist der Kapillarenkörper 10 mit einer Durchlassöffnung 14 versehen.

In dem Kapillarenkörper 10 ist ein in nur einer Richtung offener Durchlass durch ein Rückschlagventil gebildet. Das Rückschlagventil weist eine Ventilkugel 11 auf, die in bekannter Weise mittels einer Ventilfeder 12 in ihren Sitz innerhalb des Kapillarenkörpers 10 gepresst wird. Die Ventilfeder 12 wiederum ist an einem Ventilverschluss 13 abgestützt.

Zwischen der distalen Stimfläche des Antriebskolbens 5 und einer distalen Stimfläche des Abtriebskolbens 6 ist ein fluiddicht durch diese beiden Kolben 5 und 6 abgeschlossener Fluidraum mit einem ersten Teilraum 21 und einem zweiten Teilraum 22 gebildet. Die beiden Teilräume 21 und 22 sind durch den Kapillarenkörper 10 voneinander getrennt. Der Fluidraum 21, 22 ist mit einem inkompressiblen Arbeitsfluid vollkommen gefüllt. Als Arbeitsfluid wird vorzugsweise ein hochviskoses Öl verwendet.

Das Rückschlagventil 11, 12, 13 erlaubt einen Durchfluss des Arbeitsfluids nur von dem Teilraum 22 in den Teilraum 21 und verhindert einen Durchfluss in die andere Richtung.

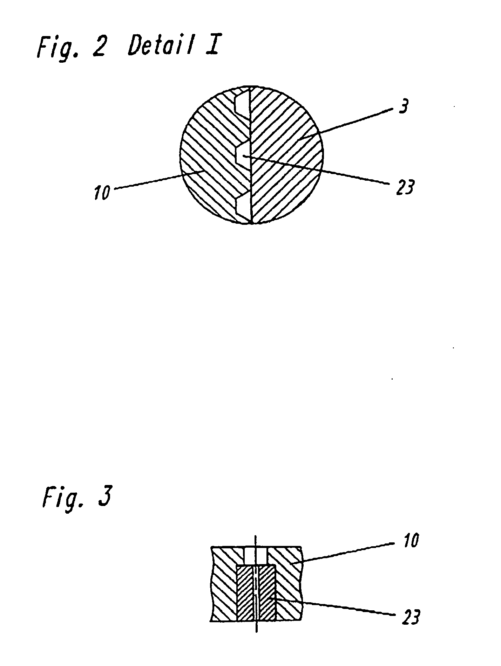

Der Kapillarenkörper 10 bildet mit einer ihn umschließenden Innenmantelfläche der äußeren Hülse 3 eine Fluidverbindung in Form eines Kapillarensystems. Das Kapillarensystem ist im Detail I der Figur 2 dargestellt. Es wird durch einen einzigen zusammenhängenden Fluidkanal, nämlich eine Kapillare 23, gebildet. Die Kapillare 23 läuft in Form eines mehrgängigen Gewindes spiralig um die äußere Mantelfläche des Kapillarenkörpers 10 um. Die Kapillare 23 kann grundsätzlich auch durch einen einzigen Gewindegang gebildet sein. Sie verbindet im Einbauzustand des Kapillarenkörpers 10 die beiden Fluidteilräume 21 und 22. Die der Kapillare 23 gegenüberliegende Innenmantelfläche der äußeren Hülse 3 ist einfach glatt. Der Kapillarenkörper 10 wird unter leichtem Pressdruck in die äußere Hülse 3 hineingeschoben. Die "Zähne" an der äußeren Mantelfläche des Kapillarenkörpers 10, die die einzelnen Gänge der Kapillare 23 voneinander trennen, drücken im eingebauten Zustand fluiddicht gegen die Innenmantelfläche der äußeren Hülse 3. Die Zähne des Kapillarenkörpers 10 sind zu Dichtzwecken abgeflacht. Der Kapillarenkörper 10 besteht aus einem weicheren Material als die äußere Hülse 3, um die Abdichtung zu verbessern. Grundsätzlich könnte jedoch zum gleichen Zweck auch die äußere Hülse 3 aus einem weicheren Material als der Kapillarenkörper 10 gefertigt sein.

In Figur 3 ist eine alternative Ausgestaltung einer Kapillare 23 dargestellt. Die Kapillare 23 wird in diesem Fall in einem Einsatzstück als gerader Fluidkanal ausgebildet. Das Einsatzstück wird in einer Aufnahme des Kapillarenkörpers fluiddicht gehalten. In dem Kapillarenkörper 10 ist eine die Kapillare 23 des Einsatzstücks verlängernde Bohrung ausgebildet, so dass auch in diesem Ausführungsbeispiel mittels einer Kapillare 23 eine Fluidverbindung zwischen den beiden Teilräumen 21 und 22 geschaffen wird.

Durch das Einsetzen eines Distanzrings 9a ist eine einfache Kompensation von sämtlichen in der Übertragungsstrecke von der Druckfeder 8 bis zu dem Abtriebskolben 6 auftretenden Abweichungen von entsprechenden Sollwerten möglich. So können mittels des Distanzrings 9a nicht nur Unterschiede bei den Druckfedern, sondern beispielsweise auch Kapillarenfehler kompensiert werden. Die Kompensation erfolgt durch Einstellung der Vorspannkraft der Druckfeder 8 mittels des einfach austauschbaren Distanzrings 9a. Der Distanzring 9a liegt für einen Gerätetyp daher in unterschiedlichen Stärken vor, und es wird bei der Montage des Geräts der Distanzring eingesetzt, der die für die Kompensation optimale Stärke aufweist.

Die Funktionsweise des Infusionsgeräts wird nachfolgend beschrieben:

In dem in Figur 1 dargestellten Zustand ist das Behältnis 1 mit dem Produkt gefüllt und der Förderkolben 2 nimmt dementsprechend seine distale Position in dem Behältnis 1 ein. Entsprechend nimmt auch der Abtriebskolben 6 seine distale Position in der inneren Hülse 4b ein. In dieser distalen Position schließt der Abtriebskolben 6 idealerweise mit der hinteren Stirnfläche der inneren Hülse 4b ab, um die Baulänge der Vorrichtung insgesamt so kurz als möglich zu halten.

Der Fluidteilraum 22 weist in diesem Zustand der Vorrichtung sein kleinstes Volumen auf. Dementsprechend weist der Fluidteilraum 21 sein grösstes Volumen auf. Der Abtriebskolben 6 wird in seiner distalen Stellung entweder unmittelbar von dem Benutzer oder vorzugsweise mittels einer Arretierung gehalten. Gleichzeitig nimmt der Antriebskolben 5 seine proximale Position ein. Die Druckfeder 8 ist in dieser proximalen Position des Antriebskolben 5 zwischen den beiden Flächen, gebildet durch die Schulterfläche des Antriebskolbens 5 und den Distanzring 9a, gespannt.

Für eine subkutane Verabreichung des Produkts wird nach dem Einstechen einer an dem proximalen Ende des Katheters 20 angebrachten Injektionsnadel die Arretierung des Abtriebskolbens 6 bzw. der Kolbenstange 7 gelöst. Unter dem Druck der Druckfeder 8 wird durch den Antriebskolben 5 im Fluidteilraum 21 ein Fluiddruck aufgebaut. Dieser Fluiddruck kann ausschließlich durch die Kapillare 23 abgebaut werden. Unter dem Druck des Antriebskolbens 5 fließt Fluid aus dem Fluidteilraum 21 durch die Kapillare 23 hindurch in den Fluidteikaum 22. Durch den sich aufbauenden Druck im Fluidteilraum 22 wird der Abtriebskolben 6 in die proximale Richtung verschoben. Der Fluidteilraum 21 bildet somit eine Antriebsseite und der Fluidteilraum 22 eine Abtriebsseite des Fluidraums 21, 22 insgesamt. Genauer gesagt werden die Antriebsseite durch eine dem Fluidteilraum 21 zugewandte Kolbenfläche des Antriebskolbens 5 und die Abtriebsseite durch eine dem Fluidteilraum 22 zugewandte Kolbenfläche des Abtriebskolbens 6 gebildet.

Eine Druckreduziereinrichtung wird im Ausführungsbeispiel durch den Kapillarenkörper 10, die äußere Hülse 3 und die im Zusammenwirken gebildete Kapillare 23 ausgebildet. Durch diese Druckreduziereinrichtung wird ein konstruktiv vorgegebener Druckabfall bewirkt. Aufgrund des erzeugten Druckabfalls ist es möglich, eine stärkere Druckfeder 8 für den Antrieb des Förderkolbens 2 zu verwenden als dies bei einem ungedrosselten Antrieb möglich wäre.

Darüber hinaus ist die Kolbenfläche des Antriebskolbens 5 größer als die Kolbenfläche des Abtriebskolbens 6. Dementsprechend bewirkt ein Hub des Antriebskolbens 5 einen demgegenüber größeren Hub des Abtriebskolbens 6. Der Abtriebskolben 6 wiederum wirkt unmittelbar mittels der steifen Kolbenstange 7 auf den Förderkolben 2. Ein voller Hub des Abtriebskolben 6 entspricht dementsprechend dem Hub des Förderkolbens 2. Der Hub des Förderkolbens 2 wiederum ist durch die üblicherweise verwendeten Behältnisse 1 vorgegeben. Dem vollen Arbeitshub des Förderkolbens 2, der einer vollständigen Ausschüttung des Inhalts des Behältnisses 1 entspricht, steht ein im Vergleich dazu wesentlich kürzerer Arbeitshub des Antriebskolbens 5 und damit der Druckfeder 8 gegenüber.

Konstruktiv interessant ist auch die konzentrische Anordnung der beiden Fluidteilräume 21 und 22 des Fluidgesamtraums 21, 22. Durch diese Anordnung kann die Baulänge der Vorrichtung insgesamt kurz gehalten werden.

Der Förderkolben 2 wird für seinen eigenen Antrieb mit einem Druck von etwa ein bar belastet, d.h. er übt solch einen Druck auf den Inhalt des Behältnisses 1 aus. Die Fluidkopplung ist entsprechend ausgebildet, um die Kraft der Druckfeder 8 von der Antriebsseite des Fluidraums 21, 22 auf die Abtriebsseite zu übertragen. Dies erfolgt im wesentlichen durch die Druckreduziereinrichtung, die durch die äußere Hülse 3, den Kapillarenkörper 10 und die Kapillare 23 gebildet wird, und durch das Größenverhältnis der beiden Kolbenflächen der Kolben 5 und 6.

Nach der Ausschüttung von Produkt, beispielsweise nach einer vollkommenen Entleerung, kann das Behältnis 1 für eine erneute Verabreichung wieder aufgefüllt oder vorzugsweise gegen ein gefülltes, neues Behältnis ausgetauscht werden. Vor dem Austausch wird der Förderkolben 2 mittels der Kolbenstange 7 in die in Figur 1 gezeigte Ausgangsstellung zurückgezogen. In der Ausgangsstellung wird die Kolbenstange 7 durch ein geeignetes Blockiermittel arretiert. Im Verlaufe des Zurückziehens drückt der Abtriebskolben 6 das Fluid aus dem vollständig gefüllten Fluidteilraum 22 in den Fluidteilraum 21. Dabei strömt das Fluid aus dem Innenraum der inneren Hülse 4b durch die Öffnung 14 in dem Boden des Kapillarenkörpers 10 und über einen kleinen Zwischenraum zwischen der Verschlusskappe 19 und dem Kapillarenkörper 10 zu dem Rückschlagventil 11, 12, 13. Unter dem Druck des Fluids in dem Fluidteilraum 22 öffnet das Rückschlagventil, und das Fluid strömt durch den mittels des Rückschlagventils gebildeten Durchfluss in den Fluidteilraum 21. Hierbei ist der Druck der Druckfeder 8 zu überwinden, um den Antriebskolben 5 in die proximale Richtung und schließlich in die gezeigte Ausgangsstellung vorzuschieben. Die Vorrichtung ist nun bereit für eine erneute Produktausschüttung.

### Bezugszeichenliste

- 1: Behältnis, Ampulle
- 2: Fördermittel, Förderkolben
- 3: Gehäuse, äußere Hülse
- 4a: Behältnishalter
- 4b: innere Hülse
- 5: Antriebskolben
- 6: Abtriebskolben
- 7: Kolbenstange
- 8: Antriebsmittel, Antriebsfeder, Druckfeder
- 9: Verschlussstück
- 9a: Distanzstück
- 10: Trennkörper, Kapillarenkörper
- 11: Ventilkugel
- 12: Ventilfeder
- 13: Ventilverschluss
- 14: Durchlassöffnung
- 15: Dichtringe
- 16: Dichtringe
- 17: Dichtringe
- 18: Dichtring
- 19: Verschlusskappe
- 20: Katheter
- 21: Fluidteilraum
- 22: Fluidteilraum
- 23: Fluidverbindung, Kapillarensystem, Fluidkanal, Kapillare

## Patentansprüche

1. Vorrichtung zur Verabreichung eines injizierbaren Produkts, umfassend
a) ein Gehäuse (3),
b) ein von dem Gehäuse (3) aufgenommenes Behältnis (1) für das Produkt,
c) ein Fördermittel (2) zur Förderung von Produkt aus dem Behältnis (1),
d) ein Antriebsmittel (8) und
e) eine Übertragungsstrecke, über die das Antriebsmittel (8) das Fördermittel (2) antreibt,
f) wobei in der Übertragungsstrecke ein Fluidraum (21, 22) für ein inkompressibles Fluid
g) und eine Druckreduziereinrichtung (3, 10, 23) vorgesehen sind, und
h) wobei der Fluidraum (21, 22) an einer Antriebsseite von dem Antriebsmittel (8) mit Druck beaufschlagbar ist und die Druckreduziereinrichtung (3, 10, 23) einen von dem Antriebsmittel (8) erzeugten Fluiddruck zu einer Abtriebsseite des Fluidraums (21, 22) hin mindert,
i) und der Fluidraum (21, 22) in einen die Antriebsseite umfassenden ersten Teilraum (21) und einen die Abtriebsseite umfassenden zweiten Teilraum (22) unterteilt ist
**dadurch gekennzeichnet, dass**
j) der erste Teilraum oder der zweite Teilraum als Ringkammer zwischen einer äußeren Hülse (3) und einer inneren Hülse (4b) und der andere der beiden Teilräume (21, 22) in der inneren Hülse (4b) ausgebildet ist.

2. Vorrichtung nach Anspruch 1, **dadurch gekennzeichnet, dass** in dem Fluidraum (21, 22) ein Arbeitshub des Antriebsmittels (8) in einen Arbeitshub des Fördermittels (2) übertragen wird, der größer als der Arbeitshub des Antriebsmittels (8) ist.

3. Vorrichtung nach dem vorhergehenden Anspruch, **dadurch gekennzeichnet, dass** eine Vorspannkraft des Antriebsmittels (8) durch ein austauschbar angeordnetes Distanzstück (9a) bestimmt wird.

4. Vorrichtung nach einem der vorhergehenden Ansprüche, **dadurch gekennzeichnet, dass** die Antriebsseite des Fluidraums (21, 22) von einer Kolbenfläche eines Antriebskolbens (5) gebildet wird, die größer ist als eine Kolbenfläche eines Abtriebskolbens (6), die die Abtriebsseite des Fluidraums (21, 22) bildet.

5. Vorrichtung nach einem der vorhergehenden Ansprüche, **dadurch gekennzeichnet, dass** die beiden Teilräume (21, 22) durch eine von der Druckreduziereinrichtung (3, 10, 23) ausgebildete Fluidverbindung (23) miteinander verbunden sind.

6. Vorrichtung nach dem vorhergehenden Anspruch, **dadurch gekennzeichnet, dass** die beiden Teilräume (21, 22) ausschließlich durch ein Kapillarensystem (23) miteinander verbunden sind, falls in dem ersten Teilraum (21) ein höherer Druck als in dem zweiten Teilraum (22) herrscht.

7. Vorrichtung nach einem der beiden vorhergehenden Ansprüche, **dadurch gekennzeichnet, dass** die Fluidverbindung (23) einen spiraligen Fluidkanal umfasst oder durch einen solchen gebildet wird.

8. Vorrichtung nach dem vorhergehenden Anspruch, **dadurch gekennzeichnet, dass** die Druckreduziereinrichtung (3, 10, 23) einen Kapillarenkörper (10) aufweist und der spiralige Fluidkanal zwischen einer Mantelfläche des Kapillarenkörpers (10) und einer Gegenmantelfläche ausgebildet ist.

9. Vorrichtung nach einem der vorhergehenden Ansprüche, **dadurch gekennzeichnet, dass**
- die Ringkammer den ersten Teilraum (21) bildet
- und ein von der äußeren Hülse (3) und der inneren Hülse (4b) fluiddicht gleitgeführter Antriebskolben (5) die Antriebsseite bildet.

10. Vorrichtung nach einem der vorhergehenden Ansprüche, **dadurch gekennzeichnet, dass**
- der zweite Teilraum (22) in der inneren Hülse (4b) gebildet ist
- und das ein fluiddicht von der inneren Hülse (4b) gleitgeführter Abtriebskolben (6) die Abtriebsseite bildet.

11. Vorrichtung nach einem der vorhergehenden Ansprüche, **dadurch gekennzeichnet, dass**
- die Druckreduziereinrichtung (3, 10, 23) einen Trennkörper (10) aufweist, der eine Stirnseite der Ringkammer bildet und die beiden Teilräume (21, 22) voneinander trennt,
- in dem Trennkörper (10) ein Ventil (11, 12, 13) aufgenommen ist, das einen Fluidfluss nur von der Abtriebsseite zu der Antriebsseite des Fluidraums (21, 22) zulässt
- und dass der Trennkörper (10) die Fluidverbindung (23) ausbildet.

## Claims

1. A device for administering an injectable product, comprising:
a) a casing (3);
b) a container (1) for said product, accommodated by said casing (3);
c) a delivering means (2) for delivering product out of said container (1);
d) a drive means (8); and
e) a transmission link via which said drive means (8) drives said delivering means (2);
f) wherein a fluid space (21, 22) for an incompressible fluid
g) and a pressure reducing means (3, 10, 23) are provided in said transmission link; and
h) wherein said fluid space (21, 22) can be impinged on a drive side by pressure from said drive means (8), and said pressure reducing means (3, 10, 23) reduces a fluid pressure generated by said drive means (8) toward a driven side of said fluid space (21, 22);
i) and said fluid space (21, 22) is sub-divided into a first partial space (21) including said drive side and a second partial space (22) including said driven side;
**characterised in that**
j) said first partial space or said second partial space is formed as a toroidal chamber between an outer sleeve (3) and an inner sleeve (4b), and the other of said two partial spaces (21, 22) is formed in said inner sleeve (4b).

2. The device as set forth in claim 1, **characterised in that** a working stroke of said drive means (8) is transmitted in said fluid space (21, 22) into a working stroke of said delivering means (2) which is greater than the working stroke of said drive means (8).

3. The device as set forth in the preceding claim, **characterised in that** a bias of said drive means (8) is determined by a replaceably arranged distance ring (9a).

4. The device as set forth in any one of the preceding claims, **characterised in that** said drive side of said fluid space (21, 22) is formed by a piston area of a drive piston (5) which is larger than a piston area of a driven piston (6) which forms the driven side of said fluid space (21, 22).

5. The device as set forth in any one of the preceding claims, **characterised in that** said two partial spaces (21, 22) are connected to each other by a fluid connection (23) formed by said pressure reducing means (3, 10, 23).

6. The device as set forth in the preceding claim, **characterised in that** said two partial spaces (21, 22) are connected to each other exclusively by a system of capillaries (23), if a higher pressure prevails in said first partial space (21) than in said second partial space (22).

7. The device as set forth in any one of the preceding two claims, **characterised in that** said fluid connection (23) includes a spiral fluid channel or is formed by the same.

8. The device as set forth in the preceding claim, **characterised in that** said pressure reducing means (3, 10, 23) comprises a capillary body (10), and said spiral fluid channel is formed between a surface area of said capillary body (10) and an opposite surface area.

9. The device as set forth in any one of the preceding claims, **characterised in that**:
- said toroidal chamber forms said first partial space (21);
- and a drive piston (5) guided fluid-proof by said outer sleeve (3) and said inner sleeve (4b) forms said drive side.

10. The device as set forth in any one of the preceding claims, **characterised in that**:
- said second partial space (22) is formed in said inner sleeve (4b);
- and said driven piston (6) guided fluid-proof by said inner sleeve (4b) forms said driven side.

11. The device as set forth in any one of the preceding claims, **characterised in that**:
- said pressure reducing means (3, 10, 23) comprises a separating body (10) which forms a front face of said toroidal chamber and separates said two partial spaces (21, 22) from each other;
- a valve (11, 12, 13) is accommodated in said separating body (10), said valve only allowing a flow of fluid from said driven side to said drive side of said fluid space (21, 22);
- and **in that** said separating body (10) forms said fluid connection (23).

## Revendications

1. Dispositif pour administrer un produit injectable, comprenant :
a) un boîtier (3),
b) un récipient (1) pour le produit, reçu par le boîtier (3),
c) un moyen de convoyage (2) pour convoyer le produit hors du récipient (1),
d) un moyen d'entraînement (8), et
e) une ligne de transmission via laquelle le moyen d'entraînement (8) entraîne le moyen de convoyage (2),
dans lequel sont prévus, dans la ligne de transmission,
f) une chambre à fluide (21, 22) pour un fluide incompressible, et
g) un dispositif de réduction de pression (3, 10, 23), et
h) dans lequel la chambre à fluide (21, 22) peut être alimentée en pression sur un côté entraînement par le moyen d'entraînement (8) et le dispositif de réduction de pression (3, 10, 23) réduit une pression de fluide engendrée par le moyen d'entraînement (8) vers un côté entraîné de la chambre à fluide (21, 22)
i) et la chambre à fluide (21, 22) est subdivisée en une première chambre partielle (21) qui comprend le côté entraînement, et une seconde chambre partielle (22) qui comprend le côté entraîné,
**caractérisé en ce que**
g) la première chambre partielle ou la seconde chambre partielle est réalisée sous forme d'une chambre annulaire entre une douille extérieure (3) et une douille intérieure (4b), et l'autre des deux chambres partielles (21, 22) est réalisée dans la douille intérieure (4b).

2. Dispositif selon la revendication 1, **caractérisé en ce que**, dans la chambre à fluide (21, 22), une course de travail du moyen d'entraînement (8) est convertie en une course de travail du moyen de convoyage (2) qui est supérieure à la course de travail du moyen d'entraînement (8).

3. Dispositif selon la revendication précédente, **caractérisé en ce qu'**une force de précontrainte du moyen d'entraînement (8) est déterminée par une pièce d'écartement (9a) agencée de manière interchangeable.

4. Dispositif selon l'une des revendications précédentes, **caractérisé en ce que** le côté entraînement de la chambre à fluide (21, 22) est formé par une surface de piston d'un piston d'entraînement (5) qui est supérieure à une surface de piston d'un piston entraîné (6) qui forme le côté entraîné de la chambre à fluide (21, 22).

5. Dispositif selon l'une des revendications précédentes, **caractérisé en ce que** les deux chambres partielles (21, 22) sont reliées l'une à l'autre par une liaison fluidique (23) réalisée par le dispositif de réduction de pression (3, 10, 23).

6. Dispositif selon la revendication précédente, **caractérisé en ce que** les deux chambres partielles (21, 22) sont reliées l'une à l'autre exclusivement par un système capillaire (23), dans le cas où il règne dans la première chambre partielle (21) une pression supérieure à celle dans la seconde chambre partielle (22).

7. Dispositif selon l'une des deux revendications précédentes, **caractérisé en ce que** la liaison fluidique (23) comprend un canal à fluide spiralé, ou est formée par un tel canal.

8. Dispositif selon la revendication précédente, **caractérisé en ce que** le dispositif de réduction de pression (3, 10, 23) comprend un corps capillaire (10), et **en ce que** le canal à fluide spiralé est formé entre une surface enveloppe du corps capillaire (10) et une surface enveloppe antagoniste.

9. Dispositif selon l'une des revendications précédentes, **caractérisé en ce que** :
-- la chambre annulaire forme la première chambre partielle (21), et
-- un piston d'entraînement (5) guidé en coulissement et de façon étanche aux fluides par la douille extérieure (3) et par la douille intérieure (4b) forme le côté entraînement.

10. Dispositif selon l'une des revendications précédentes, **caractérisé en ce que** :
-- la seconde chambre partielle (22) est formée dans la douille intérieure (4b), et
-- un piston entraîné (6) guidé en coulissement et de façon étanche aux fluides par la douille intérieure (4b) forme le côté entraîné.

11. Dispositif selon l'une des revendications précédentes, **caractérisé en ce que** :
-- le dispositif de réduction de pression (3, 10, 23) comprend un corps séparateur (10) qui forme une face frontale de la chambre annulaire et qui sépare les deux chambres partielles (21, 22) l'une de l'autre,
-- dans le corps séparateur (10) est reçue une valve (11, 12, 13) qui permet un écoulement du fluide uniquement depuis le côté entraîné vers le côté entraînement de la chambre à fluide (21, 22), et
-- le corps séparateur (10) réalise la liaison fluidique (23).
